Europäisches Patentamt

European Patent Office

Office européen des brevets

(19)

(11) Veröffentlichungsnummer: **0 098 345**
**B1**

(12) **EUROPÄISCHE PATENTSCHRIFT**

(45) Veröffentlichungstag der Patentschrift:
23.01.85

(51) Int. Cl.⁴: **C 07 C 43/29**, C 07 C 41/22,
C 07 C 41/30, C 08 K 5/06

(21) Anmeldenummer: 83102057.3

(22) Anmeldetag: 03.03.83

(54) **Tetrakis-(brommethyl)-diphenylether, Verfahren zu seiner Herstellung und seine Verwendung als Brandschutzmittel.**

(30) Priorität: 20.04.82 DE 3214416

(43) Veröffentlichungstag der Anmeldung:
18.01.84 Patentblatt 84/3

(45) Bekanntmachung des Hinweises auf die Patenterteilung:
23.01.85 Patentblatt 85/4

(84) Benannte Vertragsstaaten:
BE FR GB IT NL

(56) Entgegenhaltungen:
DE - A - 2 707 232
DE - A - 3 036 554
DE - B - 2 027 162
US - A - 3 275 708

(73) Patentinhaber: Chemische Fabrik Kalk GmbH, Kalker
Hauptstrasse 22 Postfach 91 01 57, D-5000 Köln 91 (DE)

(72) Erfinder: Jenkner, Herbert, Dr., Dipl.-Chem., Am
Quechenhauf 8, D-5024 Pulheim 2 (DE)
Erfinder: Büttgens, Walter, Petersbergstrasse 2,
D-5340 Bad Honnef (DE)

## Beschreibung

Gegenstand der vorliegenden Erfindung ist Tetrakis-(brom-methyl)-diphenyläther, seine Herstellung und seine Verwendung als Brandschutzkomponente in Kunststoffschäumen, wie Polystyrolschäumen.

Die Verwendung von organischen Bromverbindungen als Brandschutzkomponenten für Kunststoffe ist hinreichend bekannt. Vielfach ist jedoch bei der Herstellung von organischen Bromverbindungen durch Bromierung entsprechender Kohlenwasserstoffe der Umgang mit elementarem Brom unumgänglich, der durch die Aggressivität des Broms besondere technische Aufwendungen und Schutzmaßnahmen notwendig macht.

Darüber hinaus fällt bei Bromierungen organischer Verbindungen mit elementarem Brom als Nebenprodukt Bromwasserstoff an, der bei großtechnischen Bromierungen in einer Wasservorlage aufgefangen und als wäßrige Bromwasserstoffsäure einer Wiederverwendung zugeführt werden kann. In anderen Fällen kann es vorteilhaft sein, den Bromwasserstoff in Eisessig aufzufangen und in dieser Form für weitere chemische Umsetzungen zu verwenden.

Es ergab sich hieraus die Aufgabe, eine Verwendungsmöglichkeit für derartige Lösungen von Bromwasserstoff in Eisessig zu finden, die zu einer als Brandschutzkomponente für verschäumte Kunststoffe, insbesondere verschäumtes Polystyrol, geeigneten organischen Bromverbindung führt.

Gegenstand der Erfindung ist Tetrakis-(brommethyl)-diphenyläther. Zu seiner Herstellung wird vorteilhaft Diphenyläther unter Einsatz von in Eisessig gelöstem Bromwasserstoff mit Formaldehyd, in Form von Paraformaldehyd, umgesetzt. Das erfindungsgemäße Produkt hat einen Schmelzbereich von 118—120°C und ist verwendbar als Brandschutzkomponente für Kunststoffschäume, insbesondere für Polystyrolschäume.

Nach einem in der DE-PS 20 27 162 beschriebenen Verfahren werden Benzolkohlenwasserstoffe, u. a. auch Diphenyläther, in ähnlicher Weise mit Fomaldehyd und Bromwasserstoff in einer aliphatischen Carbonsäure gelöst zu Brommethylgruppen enthaltenden Verbindungen umgesetzt. Vornehmlich werden jedoch nach diesem Verfahren der DE-PS 20 27 162 Methylgruppen enthaltende Verbindungen zur Brommethylierung verwendet, und es war überraschend, festzustellen, daß nach dem erfindungsgemäßen Verfahren der nicht substituierte Diphenyläther mit 4 Brommmethylgruppen substituiert werden kann.

Zur Herstellung des erfindungsgemäßen Tetrakis-(brommethyl)-diphenyläthers wird Diphenyläther in Eisessig vorgelegt und die 2fache äquimolare Menge Paraformaldehyd sowie die 2fache äquimolare Menge Bromwasserstoff in Form einer 35,2%igen HBr/Eisessiglösung zugefügt. Die hier insgesamt einzusetzende Menge Eisessig entspricht etwa der 10fachen Menge an Diphenyläther. Die Umsetzung wird in einer mehrstündigen Reaktionszeit bei einer Reaktionstemperatur von 90°C unter Rühren durchgeführt. Die Reaktionspartner gehen dabei vollständig in Lösung. Nach beendeter Reaktionszeit und anschließendem Abkühlen des Gemisches auf Raumtemperatur fällt der Tetrakis-(brommethyl)-diphenyläther als schwach gefärbtes feinteilig kristallisiertes Produkt aus. Nach dem Abtrennen des Produktes und anschließendem Digerieren bzw. mehrmaligem Waschen mit Wasser bis zur neutralen Reaktion wird das Produkt getrocknet. Es wird in einer Ausbeute von 94,5%, bezogen auf eingesetzten Diphenyläther, erhalten und hat einen Schmelzbereich von 118—120°C mit einem nahezu theoretischen Bromgehalt von 58,5%.

In Fällen, wo keine HBr/Eisessiglösung zur Herstellung von Tetrakis-(brommethyl)-diphenyläther zur Verfügung steht, ist es ebenso und mit gleichem Erfolg möglich, die Reaktion unter gleichen Bedingungen bei kontinuierlichem Einleiten von Bromwasserstoffgas in das Reaktionsgemisch aus Eisessig, Diphenyläther und Paraformaldehyd durchzuführen.

Der erfindungsgemäße Tetrakis-(brommethyl)-diphenyläther ist vielseitig verwendbar, wie beipielsweise als Reaktionspartner bei der Umsetzung mit Phosphiten. Dabei werden nach der nach Arbusow genannten Reaktion Phosphonsäureester erhalten, die, falls nicht alle Brommethylgruppen bei einer solchen Reaktion ersetzt werden, auch noch Brom am Molekül enthalten können. Ebenso bietet sich die Möglichkeit, den Tetrakis-(brommethyl)-diphenyläther als Zwischenprodukt für die Herstellung von Methylolverbindungen, Cyaniden oder Thioverbindungen einzusetzen. Insbesondere ist Tetrakis-(brommethyl)-diphenyläther als Brandschutzkomponente für Schaum-Kunststoffe, wie beispielsweise Polystyrolschäume geeignet.

In den folgenden Beispielen wird die Herstellung des erfindungsgemäßen Tetrakis-(brommethyl)-diphenyläthers sowie dessen Verwendung als Brandschutzkomponente für einen Schaum-Kunststoff erläutert.

Beispiel 1 (Herstellung)

In einem Reaktionsgefäß, das mit Rührer, Thermometer und Rückflußkühler versehen ist, werden 170 Gew.-Teile Diphenyläther und 300 Gew.-Teile Eisessig vorgelegt, und 240 Gew.-Teile Paraformaldehyd und 2000 Gew.-Teile einer 32,5%igen HBr/Eisessiglösung werden zugefügt. Das Gemisch wird unter Rühren auf einer Temperatur von 90°C erwärmt, wobei die Reaktionspartner vollständig in Lösung gehen. Nach einer 10stündigen Reaktionszeit bei einer Temperatur von 90°C beginnt sich das Umsetzungsprodukt allmählich als fein kristallisierter Niederschlag abzuscheiden. Die Reaktion wird weitere 14 Stunden bei gleicher Temperatur fortgesetzt, und nach einer Reaktionszeit von insgesamt 24 Stunden bei 90° C wird das Gemisch auf Raumtemperatur abgekühlt. Der hierbei ausgefallene kristalline Tetrakis-(brommethyl)-diphenyläther wird abfiltriert, in 2000 Gew.-Teilen Wasser aufgeschlämmt und diese Aufschlämmung 2 Stunden bei Raumtemperatur gerührt. Danach wird das Produkt wieder abfiltriert und bis zur neutralen Reaktion mehrmals mit Wasser gewaschen. Nach dem Trocknen bei einer Temperatur von 80° C werden 512 Gew.-Teile Tetrakis-(brommethyl)-diphenyläther als schwach gefärbtes, kristallines Produkt mit einem Schmelzbereich von 118—120° C erhalten. Die Ausbeute beträgt 94,5% der Theorie, bezogen auf eingesetzten Diphenyläther. Der Bromgehalt entspricht mit 58,8% Brom nahezu dem theoretischen Wert.

Beispiel 2 (Verwendung)

Auf 100 Gew.-Teile Polystyrol-Schaumgranalien (EPS) mit einem Raumgewicht von 40 g/l werden vor dem Verschäumen 1,2 Gew.-Teile bzw. 1,6 Gew.-Teile Tetrakis-(brommethyl)-diphenyläther mittels eines Haftvermittlers aufgetrommelt. Nach dem Verschäumen der Granalien werden aus dem Schaum Formteile von 152 × 51 × 13 mm Seitenlänge geschnitten und damit das Brandverhalten entsprechend dem Brandtest ASTM 1692-74 geprüft.

Dabei ergeben sich folgende Werte:

| Tetrakis-(brommethyl)-diphenyläther | Selbstver-löschung nach sec | Abbrand-strecke mm |
|---|---|---|
| a) 1,2 Gew.-% auf EPS | 37 | 52 |
| b) 1,6 Gew.-% auf EPS | 26 | 45 |

**Patentansprüche**

1. Tetrakis-(brommethyl)-diphenyläther.

2. Verfahren zur Herstellung von Tetrakis-(brommethyl)-diphenyläther nach Anspruch 1, dadurch gekennzeichnet, daß Diphenyläther unter Einsatz von in Eisessig gelöstem Bromwasserstoff mit Formaldehyd, in Form von Paraformaldehyd, bei einer Temperatur von 90° C umgesetzt wird.

3. Verwendung des Produktes nach Ansprüchen 1 und 2 als Brandschutzkomponente für Kunststoffschäume, insbesondere für Polystyrolschäume.

**Claims**

1. Tetrakis-(bromomethyl)-diphenyl ether.

2. Process for the preparation of tetrakis-(bromomethyl)-diphenyl ether according to Claim 1, characterised in that diphenyl ether is made to react with formaldehyde, in the form of paraformaldehyde, at a temperature of 90° C using hydrogen bromide dissolved in glacial acetic acid.

3. Use of the product according to Claims 1 and 2 as a fire-proofing agent for plastic foams, in particular polystyrene foams.

**Revendications**

1. Tetrakis-(brommométhyl)-diphényl éther.

2. Procédé de préparation de tetrakis-(bromo-méthyl)-diphényl éther suivant la revendication 1, caractérisé en ce que du diphényl éther est mis à réagir, en présence d'acide bromhydrique dissous dans de l'acide acétique glacial, avec du formaldéhyde sous forme de paraformaldéhyde, à une température de 90° C.

3. Utilisation du produit suivant les revendications 1 et 2 comme agent ignifugeant pour mousses de matières plastiques, notamment pour mousses de polystyrène.